# EUROPÄISCHE PATENTSCHRIFT

(11) **EP 3 383 839 B1**
(45) Veröffentlichungstag und Bekanntmachung des Hinweises auf die Patenterteilung: **06.05.2020**
(21) Anmeldenummer: 16805436.9
(22) Anmeldetag: 02.12.2016
(51) Int. Cl.: C07C 257/14, C07C 257/16, C07C 257/18

(54) **VERFAHREN ZUR HERSTELLUNG VON N,N´-UNSUBSTITUIERTEN CARBOXAMIDINEN**
PROCESS FOR THE PREPARATION OF N,N´-UNSUBSTITUTED CARBOXAMIDINES
PROCÉDÉ DE PRODUCTION DE N,N'-CARBOXAMIDINES NON SUBSTITUÉES

(30) Priorität: 04.12.2015 DE 102015015692
(43) Veröffentlichungstag der Anmeldung: 10.10.2018
(73) Patentinhaber: AlzChem Trostberg GmbH, 83308 Trostberg (DE)
(72) Erfinder: GÜTHNER, Thomas, 83308 Trostberg (DE)
(74) Vertreter: Weickmann & Weickmann PartmbB
(86) Internationale Anmeldenummer: PCT/EP2016/079611
(87) Internationale Veröffentlichungsnummer: WO 2017/093495

(56) Entgegenhaltungen:
- N. DE KIMPE ET AL.: "A new and facile synthesis of trialkylketenimines", JOURNAL OF ORGANIC CHEMISTRY, Bd. 43, Nr. 13, 1. Juni 1978 (1978-06-01), Seiten 2670-2672, XP002768534, DOI: 10.1021/jo00407a025

## Beschreibung

Die vorliegende Erfindung betrifft ein neues Verfahren zur Herstellung von *N,N'*-unsubstituierten Carboxamidinen oder einem Salz hiervon. Des Weiteren betrifft die vorliegende Erfindung ein neues Verfahren zur Herstellung von *N,N'*-unsubstituierten Carboxamidinen, in dem eine Carboxamidingruppe in einem Schritt in eine gegebene Grundstruktur eingeführt wird.

*N,N'*-unsubstituierte Carboxamidine weisen die allgemeine Formel R-C(=NH)-NH₂ auf und sind wertvolle Bausteine zur Synthese von Wirkstoffen. Diese Carboxamidine weisen im Vergleich zu Aminen oder Amiden eine relativ hohe Basizität auf und bilden mit Säuren stabile Salze, in denen die positive Ladung über die gesamte funktionelle Gruppe (Carboxamidingruppe) verteilt ist. Die freien Basen werden insbesondere zur Herstellung von Pharmazeutika und Pflanzenschutzmitteln genutzt. Auch als Zwischenprodukt zur Herstellung von Heterocyclen, wie beispielsweise Pyrimidine und 1,3,5-Triazine, sind Carboxamidine sehr gefragt.

Übliche Syntheseverfahren zur Herstellung von Carboxamidinen gehen von Carbonitrilen (= Organo-Cyaniden) der Formel R-CN aus. Die Umsetzung kann entweder mit Alkoholen R'-OH und einer Mineralsäure zu Imidaten R-C(=NH)-OR' und weiter mit Ammoniak, primären Aminen oder sekundären Aminen zum gewünschten Carboxamidin R-C(=NH)-NR'R" (Pinner-Reaktion) erfolgen. *N*,*N*'-unsubstituierte Carboxamidine werden in dieser Synthese ausschließlich durch die Reaktion der Imidate mit Ammoniak erhalten. Alternativ können *N*,*N*'-unsubstituierte Carboxamidine auch durch Addition von Hydroxylamin an das Carbonitril R-CN zum *N*-Hydroxyamidin R(=N-OH)-NH₂, das seinerseits durch Reduktion in die gewünschten Carboxamidine R-C(=NH)-NH₂ überführt werden kann, erhalten werden. Beide Verfahrensvarianten sind als mehrstufige Verfahren relativ aufwändig und liefern nicht immer gute Ausbeuten. Zudem sind die als Ausgangsstoffe benötigten Carbonitrile nicht immer gut zugänglich.

N. De Kimpe et al. (Journal of Organic Chemistry, Bd. 43, Nr. 13, (1978), Seiten 2670-2672) betrifft die Herstellung von Trialkylketeniminen der Formel (R1)(R2)C-C(=NR)-NHR' (6), wobei ein α-Cyanenamin (R1)(R2)C=C(-NHR)-CN (1) mit einem MeMgl-Reagent umgesetzt wird, was zur Bildung eines Trialkylketenimins der Formel (R1)(R2)C=C=NR (2) führt, welches dann in einem weiteren Schritt mit einem primären Amin R'-NH2 umgesetzt wird, um ein Trialkylcarboxyamidin (6) zu bilden.

Der vorliegenden Erfindung liegt daher die Aufgabe zugrunde, ein neues Verfahren zur Herstellung von *N,N'*-unsubstituierten Carboxamidinen bereitzustellen, das in einfacher Art und Weise die Einführung einer Carboxamindingruppe in eine bestehende Struktur ermöglicht sowie ein Verfahren, das in breiter Form anwendbar ist und eine hohe Raum-Zeit-Ausbeute ermöglicht.

Gelöst werden diese Aufgaben durch ein Verfahren gemäß Anspruch 1. Demnach ist gemäß einer ersten Ausführungsform ein Verfahren zur Herstellung von *N,N'*-unsubstituierten Carboxamidinen der allgemeinen Formel (I) oder einem Salz hiervon Gegenstand der vorliegenden Erfindung, wobei für die Formel (I) gilt:

R¹-C(=NH)-NH₂ **Formel (I)**

wobei für den Rest R¹ gilt:
- R¹ =: linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste R¹ weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino,
umfassend die Verfahrensschritte:
a) Umsetzung einer magnesiumorganischen Verbindung der allgemeinen Formel (II) mit einem *N,N*'-Bis(trialkylsilyl)carbodiimid gemäß Formel (III) in einem organischen Lösungsmittel, wobei für Formel (II) und Formel (III) gilt:

   R¹-Mg-X **Formel (II)**

   wobei für den Rest R¹ die oben genannte Bedeutung und für den Rest X gilt:
   X = Chlor, Brom oder lod,

   (R²)₃Si-N=C=N-Si(R²)₃ **Formel (III)**

   wobei für die Reste R² gleichzeitig oder unabhängig voneinander gilt,
   R² = gleichzeitig oder unabhängig voneinander linear oder verzweigt C₁₋ bis C₅-Alkyl,
b) Hydrolysieren des in Schritt a) intermediär gebildeten, silylierten Magnesium-Carboxamidinat gemäß Formel (IV)

   R¹-C(=N-Si(R²)₃)-N(-Si(R²)₃)·Mg-X **Formel (IV)**

   wobei für die Reste R¹, R² und X die oben genannte Bedeutung gilt,
c) Isolieren der *N,N'*-unsubstituierten Carboxamidine der allgemeinen Formel (I) oder eines Salzes hiervon.

Es ist bekannt, dass Grignard-Verbindungen der Formel R-Mg-X (X = Cl, Br, J) mit Kohlendioxid glatt und in guter Ausbeute zu Carbonsäuren der Formel R-COOH reagieren. Überraschenderweise wurde nun gefunden, dass die aus wasserfreiem Cyanamid gut zugänglichen Bis-trialkylsilylcarbodiimide, insbesondere *N*,*N'-*Bis(trimethylsilyl)carbodiimid (BTSCD), mit Grignard-Reagentien eine analoge Reaktion zeigen. Die entstehenden silylierten Magnesium-Carboxamidinate der Formel R¹-C(=N-Si(R²)₃)-N(Si(R²)₃)·Mg-X hydrolysieren zudem überraschenderweise bereitwillig bei der Grignard-üblichen wässrigsauren Aufarbeitung unter Abspaltung der Silylgruppen, so dass Alkylhalogenide R¹-X in einem Eintopfverfahren direkt zu *N,N'*-unsubstituierten Carboxamidinen umgewandelt werden können.

Ohne an die Theorie gebunden zu sein, könnte das neue Verfahren zur Herstellung von *N,N'*-unsubstituierten Carboxamidinen abstrakt gemäß des folgenden Schema 1 formuliert werden. Hierbei kann als zentraler Schritt die Umsetzung der magnesiumorganischen Verbindung mit dem Bis-(trialkylsilyl)carbodiimid angesehen werden, wobei silylierte Magnesium-Carboxamidinate der Formel R¹-C(=N-Si(R²)₃)-N(Si(R²)₃)·Mg-X gebildet werden.

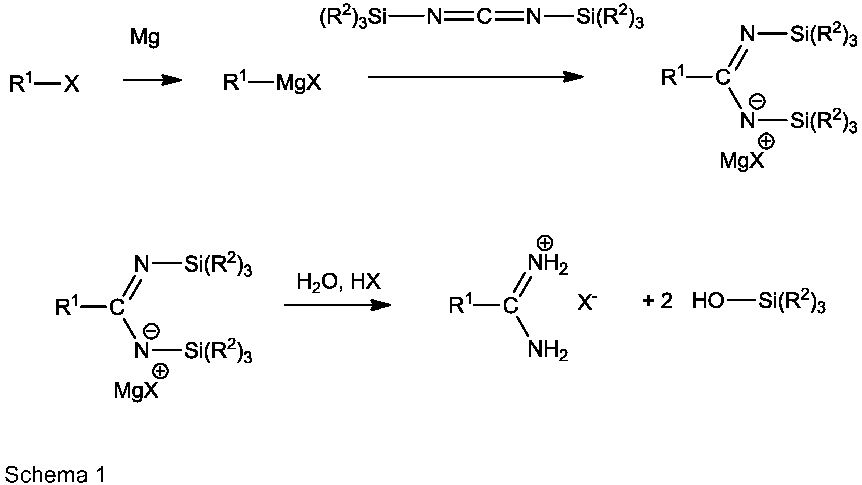

Das Verfahren gemäß der vorliegenden Erfindung lässt sich breit anwenden und ist im Prinzip für alle Reste R¹ geeignet, die keine Protonen-abspaltende oder mit Grignard-Reagentien reagierende Gruppe enthalten, also mit der Herstellung eines Grignard-Reagens kompatibel sind. Erfindungsgemäß steht R¹ daher für linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino.

Dabei soll im Zusammenhang mit der vorliegenden Erfindung unter linear oder verzweigt C₁- bis C₂₀-Alkyl ein linearer oder verzweigter Alkylrest mit 1 bis 20 Kohlenstoffatomen verstanden sein, der insbesondere die allgemeine Formel CₙH₂ₙ₊₁ aufweist, wobei n = eine ganze Zahl von 1 bis 20 bedeutet. Dabei ist insbesondere vorgesehen, dass linear oder verzweigt C₁- bis C₂₀-Alkyl Methyl, Ethyl, n-Propyl, 1-Methylethyl, n-Butyl, 1-Methylpropyl, 2-Methylpropyl, 2,2-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, n-Heptyl, 1-Methylhexyl, 2-Methylhexyl, 3-Methylhexyl, 4-Methylhexyl, 5-Methylhexyl, 1,1-Dimethylpentyl, 1,2-Dimethylpentyl, 1,3-Dimethylpentyl, 1,4-Dimethylpentyl, 2,2-Dimethylpentyl, 2,3-Dimethylpentyl, 2,4-Dimethylpentyl, 3,3-Dimethylpentyl, 3,4-Dimethylpentyl, 4,4-Dimethylpentyl, 1-Ethylpentyl, 2-Ethylpentyl, 3-Ethylpentyl, 1,2,3-Trimethylbutyl, 1,1,2-Trimethylbutyl, 1,1,3-Trimethylbutyl, 2,2,3-Trimethylbutyl, 2,3,3-Trimethylbutyl, 1-Ethyl-1-methylbutyl, 1-Ethyl-2-methylbutyl, 1-Ethyl-3-methylbutyl, 2-Ethyl-1-methylbutyl, 2-Ethyl-2-methylbutyl, 1-Ethyl-3-methylbutyl, 1-Propylbutyl, n-Octyl, 1-Methylheptyl, 2-Methylheptyl, 3-Methylheptyl, 4-Methylheptyl, 5-Methylheptyl, 6-Methylheptyl, 1,1-Dimethylhexyl, 1,2-Dimethylhexyl, 1,3-Dimethylhexyl, 1,4-Dimethylhexyl, 1,5-Dimethylhexyl, 2,2-Dimethylhexyl, 2,3-Dimethylhexyl, 2,4-Dimethylhexyl, 2,5-Dimethylhexyl, 3,3-Dimethylhexyl, 3,4-Dimethylhexyl, 3,5-Dimethylhexyl, 4,4-Dimethylhexyl, 4,5-Dimethylhexyl, 5,5-Dimethylhexyl, 1-Ethylhexyl, 2-Ethylhexyl, 3-Ethylhexyl, 4-Ethylhexyl, 1-Ethyl-1-methylpentyl, 1-Ethyl-2-methylpentyl, 1-Ethyl-3-methylpentyl, 1-Ethyl-4-methylpentyl, 2-Ethyl-1-methylpentyl, 2-Ethyl-2-methylpentyl, 2-Ethyl-3-methylpentyl, 2-Ethyl-4-methylpentyl, 3-Ethyl-1-methylpentyl, 3-Ethyl-2-methylpentyl, 3-Ethyl-3-methylpentyl, 3-Ethyl-4-methylpentyl, 1-Propylpentyl, 2-Propylpentyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosanyl bedeutet.

Weiterhin soll im Zusammenhang mit der vorliegenden Erfindung unter linear oder verzweigt C₁- bis C₅-Alkyl ein Alkylrest mit 1 bis 5 Kohlenstoffatomen verstanden sein, der insbesondere die allgemeine Formel CₙH₂ₙ+₁ aufweist, wobei n = eine ganze Zahl von 1 bis 5 bedeutet. Dabei ist insbesondere vorgesehen, dass linear oder verzweigt C₁- bis C₅-Alkyl Methyl, Ethyl, n-Propyl, 1-Methylethyl, n-Butyl, 1-Methylpropyl, 2-Methylpropyl, n-Pentyl, 1-Methylbutyl, 2-Methylbutyl, 3-Methylbutyl, 1,1-Dimethylpropyl, 1,2-Dimethylpropyl, 2,2-Dimethylpropyl oder 1-Ethylpropyl bedeutet.

Weiterhin soll im Zusammenhang mit der vorliegenden Erfindung unter C₃- bis C₂₀-Cycloalkyl ein monocyclischer Cycloalkylrest mit 3 bis 20 Kohlenstoffatomen verstanden sein, der insbesondere die allgemeine Formel CₙH₂ₙ₋₁ aufweist, oder ein bicyclischer Cycloalkylrest mit 3 bis 20 Kohlenstoffatomen verstanden sein, der insbesondere die allgemeine Formel CₙH₂ₙ₋₃ aufweist, wobei jeweils n = eine ganze Zahl von 3 bis 20 bedeutet.

Weiterhin soll im Zusammenhang mit der vorliegenden Erfindung unter C₃- bis C₁₅-Cycloalkyl ein monocyclischer Cycloalkylrest mit 3 bis 15 Kohlenstoffatomen verstanden sein, der insbesondere die allgemeine Formel CₙH₂ₙ₋₁ aufweist, oder ein bicyclischer Cycloalkylrest mit 3 bis 15 Kohlenstoffatomen verstanden sein, der insbesondere die allgemeine Formel CₙH₂ₙ₋₃ aufweist, wobei jeweils n = eine ganze Zahl von 3 bis 15 bedeutet.

Dabei ist insbesondere auch vorgesehen, dass C₃- bis C₁₅-Cycloalkyl oder C₃- bis C₂₀-Cycloalkyl Cyclopropyl, Cyclobutyl, Cyclopentyl, Cyclohexyl oder Cycloheptyl bedeuten, wobei diese Cycloalkylreste ihrerseits weiterhin bevorzugt einfach oder mehrfach mit linear oder verzweigt C₁- bis C₅-Alkyl der oben angegebenen Bedeutung substituiert sein können.

Weiterhin bevorzugt kann damit C₃- bis C₂₀-Cycloalkyl auch 1-Methyl-1-Cyclopropyl, 1-Methyl-1-Cyclobutyl, 1-Methyl-1-Cyclopentyl, 1-Methyl-1-Cyclohexyl, 1-Methyl-1-Cycloheptyl, 2-Methyl-1-Cyclopropyl, 2-Methyl-1-Cyclobutyl, 2-Methyl-1-Cyclopentyl, 2-Methyl-1-Cyclohexyl, 2-Methyl-1-Cycloheptyl, 3-Methyl-1-Cyclobutyl, 3-Methyl-1-Cyclopentyl, 3-Methyl-1-Cyclohexyl, 3-Methyl-1-Cycloheptyl, 4-Methyl-1-Cyclohexyl, 4-Methyl-1-Cycloheptyl, 1 ,2-Dimethyl-1-Cyclopropyl, 2,2-Dimethyl-1-Cyclopropyl, 2,3-Dimethyl-1-Cyclopropyl, 1 ,2-Dimethyl-1-Cyclobutyl, 1 ,3-Dimethyl-1-Cyclobutyl, 2,2-Dimethyl-1-Cyclobutyl, 2,3-Dimethyl-1-Cyclobutyl, 2,4-Dimethyl-1-Cyclobutyl, 3,3-Dimethyl-1-Cyclobutyl, 1,2-Dimethyl-1-Cyclopentyl, 1,3-Dimethyl-1-Cyclopentyl, 2,2-Dimethyl-1-Cyclopentyl, 2,3-Dimethyl-1-Cyclopentyl, 2,4-Dimethyl-1-Cyclopentyl, 2,5-Dimethyl-1-Cyclopentyl, 3,3-Dimethyl-1-Cyclopentyl, 3,4-Dimethyl-1-Cyclopentyl, 1,2-Dimethyl-1-Cyclohexyl, 1,3-Dimethyl-1-Cyclohexyl, 1,4-Dimethyl-1-Cyclohexyl, 1,5-Dimethyl-1-Cyclohexyl, 1,6-Dimethyl-1-Cyclohexyl, 2,2-Dimethyl-1-Cyclohexyl, 2,3-Dimethyl-1-Cyclohexyl, 2,4-Dimethyl-1-Cyclohexyl, 2,5-Dimethyl-1-Cyclohexyl, 2,6-Dimethyl-1-Cyclohexyl, 3,3-Dimethyl-1-Cyclohexyl, 3,4-Dimethyl-1-Cyclohexyl, 3,5-Dimethyl-1-Cyclohexyl, 3,6-Dimethyl-1-Cyclohexyl, 4,4-Dimethyl-1-Cyclohexyl, 1,2,2-Trimethyl-1-Cyclopropyl, 1,2,3-Trimethyl-1-Cyclopropyl, 1,2,2-Trimethyl-1-Cyclobutyl, 1,3,3-Trimethyl-1-Cyclobutyl, 1,2,3-Trimethyl-1-Cyclobutyl, 2,2,3-Trimethyl-1-Cyclobutyl, 2,2,4-Trimethyl-1-Cyclobutyl, 1,2,2-Trimethyl-1-Cyclopentyl, 1,2,3-Trimethyl-1-Cyclopentyl, 1,2,4-Trimethyl-1-Cyclopentyl, 1,2,5-Trimethyl-1-Cyclopentyl, 1,3,3-Trimethyl-1-Cyclopentyl, 1,3,4-Trimethyl-1-Cyclopentyl, 1,3,5-Trimethyl-1-Cyclopentyl, 2,2,3-Trimethyl-1-Cyclopentyl, 2,2,4-Trimethyl-1-Cyclopentyl, 2,2,5-Trimethyl-1-Cyclopentyl, 2,3,3-Trimethyl-1-Cyclopentyl, 2,3,4-Trimethyl-1-Cyclopentyl, 2,3,5-Trimethyl-1-Cyclopentyl, 2,3,3-Trimethyl-1-Cyclopentyl, 2,4,4-Trimethyl-1-Cyclopentyl, 2,4,5-Trimethyl-1-Cyclopentyl, 2,5,5-Trimethyl-1-Cyclopentyl, 3,3,4-Trimethyl-1-Cyclopentyl, 3,3,5-Trimethyl-1-Cyclopentyl, 3,4,5-Trimethyl-1-Cyclopentyl, 3,4,4-Trimethyl-1-Cyclopentyl, 1,2,2-Trimethyl-1-Cyclohexyl, 1,2,3-Trimethyl-1-Cyclohexyl, 1,2,4-Trimethyl-1-Cyclohexyl, 1,2,5-Trimethyl-1-Cyclohexyl, 1,2,6-Trimethyl-1-Cyclohexyl, 1,3,3-Trimethyl-1-Cyclohexyl, 1,3,4-Trimethyl-1-Cyclohexyl, 1,3,5-Trimethyl-1-Cyclohexyl, 1,3,6-Trimethyl-1-Cyclohexyl, 1,4,4-Trimethyl-1-Cyclohexyl, 2,2,3-Trimethyl-1-Cyclohexyl, 2,2,4-Trimethyl-1-Cyclohexyl, 2,2,5-Trimethyl-1-Cyclohexyl, 2,2,6-Trimethyl-1-Cyclohexyl, 2,3,3-Trimethyl-1-Cyclohexyl, 2,3,4-Trimethyl-1-Cyclohexyl, 2,3,5-Trimethyl-1-Cyclohexyl, 2,3,6-Trimethyl-1-Cyclohexyl, 2,4,4-Trimethyl-1-Cyclohexyl, 2,4,5-Trimethyl-1-Cyclohexyl, 2,4,6-Trimethyl-1-Cyclohexyl, 2,5,5-Trimethyl-1-Cyclohexyl, 2,5,6-Trimethyl-1-Cyclohexyl, 2,6,6-Trimethyl-1-Cyclohexyl, 3,3,4-Trimethyl-1-Cyclohexyl, 3,3,5-Trimethyl-1-Cyclohexyl, 3,3,6-Trimethyl-1-Cyclohexyl, 3,4,4-Trimethyl-1-Cyclohexyl, 3,4,5-Trimethyl-1-Cyclohexyl, 3,4,6-Trimethyl-1-Cyclohexyl, 3,5,6-Trimethyl-1-Cyclohexyl, 1,2,3,3-Tetramethyl-1-Cyclopropyl, 2,2,3,3-Tetramethyl-1-Cyclopropyl, 1,2,2,3-Tetramethyl-1-Cyclopropyl, 1,2,2,3-Tetramethyl-1-Cyclobutyl, 1,2,3,3-Tetramethyl-1-Cyclobutyl, 2,2,3,3-Tetramethyl-1-Cyclobutyl, 2,3,3,4-Tetramethyl-1-Cyclobutyl, 1,2,2,3-Tetramethyl-1-Cyclopentyl, 1,2,2,4-Tetramethyl-1-Cyclopentyl, 1,2,2,5-Tetramethyl-1-Cyclopentyl, 1,2,3,3-Tetramethyl-1-Cyclopentyl, 1,2,3,4-Tetramethyl-1-Cyclopentyl, 1,2,3,5-Tetramethyl-1-Cyclopentyl, 1,2,5,5-Tetramethyl-1-Cyclopentyl, 2,2,3,3-Tetramethyl-1-Cyclopentyl, 2,2,3,3-Tetramethyl-1-Cyclohexyl, 2,2,4,4-Tetramethyl-1-Cyclohexyl, 2,2,5,5-Tetramethyl-1-Cyclohexyl, 3,3,4,4-Tetramethyl-1-Cyclohexyl, 3,3,5,5-Tetramethyl-1-Cyclohexyl, 1-Ethyl-1-Cyclopropyl, 1-Ethyl-1-Cyclobutyl, 1-Ethyl-1-Cyclopentyl, 1-Ethyl-1-Cyclohexyl), 1-Ethyl-1-Cycloheptyl, 2-Ethyl-1-Cyclopropyl, 2-Ethyl-1-Cyclobutyl, 2-Ethyl-1-Cyclopentyl, 2-Ethyl-1-Cyclohexyl, 2-Ethyl-1-Cycloheptyl, 3-Ethyl-1-Cyclobutyl, 3-Ethyl-1-Cyclopentyl, 3-Ethyl-1-Cyclohexyl, 3-Ethyl-1-Cycloheptyl, 4-Ethyl-1-Cyclohexyl oder 4-Ethyl-1-Cycloheptyl bedeuten.

Weiterhin soll im Zusammenhang mit der vorliegenden Erfindung unter C₄- bis C₂₀-Cycloalkylalkyl ein linear oder verzweigt C₁- bis C₅-Alkyl der oben angegebenen Bedeutung verstanden sein, der seinerseits mit C₃- bis C₁₅-Cycloalkyl der oben angegebenen Bedeutung substituiert ist, und der insbesondere die allgemeine Formel CₙH₂ₙ₋₁ aufweist, wobei n = eine ganze Zahl von 4 bis 20 bedeutet.
Damit kann C₄- bis C₂₀-Cycloalkylalky insbesondere auch C₃- bis C₁₅-Cycloalkyl-methyl, 1-(C₃- bis C₁₅-Cycloalkyl)-1-ethyl, 2-(C₃- bis C₁₅-Cycloalkyl)-1-ethyl, 1-(C₃- bis C₁₅-Cycloalkyl)-1-propyl, 2-(C₃- bis C₁₅-Cycloalkyl)-1-propyl oder 3-(C₃- bis C₁₅-Cycloalkyl)-1-propyl bedeuten, wobei C₃- bis C₁₅-Cycloalkyl die oben wiedergegeben Beutung aufweist.

Weiterhin soll im Zusammenhang mit der vorliegenden Erfindung unter C₆- bis C₂₀-Aryl ein aromatischer Arylrest mit 6 bis 20 Kohlenstoffatomen, insbesondere ein monocyclischer aromatischer Arylrest mit 6 bis 20 Kohlenstoffatomen oder ein bicyclischer aromatischer Arylrest mit 6 bis 20 Kohlenstoffatomen oder ein tricyclischer aromatischer Arylrest mit 6 bis 20 Kohlenstoffatomen, verstanden sein, der weiterhin bevorzugt seinerseits einfach oder mehrfach mit linear oder verzweigt C₁- bis C₅-Alkyl der oben wiedergegebenen Bedeutung substituiert sein kann.

Weiterhin soll im Zusammenhang mit der vorliegenden Erfindung unter C₆- bis C₁₅-Aryl ein aromatischer Arylrest mit 6 bis 15 Kohlenstoffatomen, insbesondere einen monocyclischer aromatischer Arylrest mit 6 bis 15 Kohlenstoffatomen oder ein bicyclischer aromatischer Arylrest mit 6 bis 15 Kohlenstoffatomen oder ein tricyclischer aromatischer Arylrest mit 6 bis 15 Kohlenstoffatomen, verstanden sein, der weiterhin bevorzugt seinerseits einfach oder mehrfach mit linear oder verzweigt C₁- bis C₅-Alkyl der oben wiedergegebenen Bedeutung substituiert sein kann.

Besonders bevorzugt kann somit vorgesehen sein, dass C₆- bis C₂₀-Aryl oder C₆- bis C₁₅-Aryl ein Benzolrest (Phenyl), Naphthalinrest (Naphthyl), Anthracenrest oder Perylenrest bedeutet, der seinerseits einfach oder mehrfach mit linear oder verzweigt C₁- bis C₅-Alkyl der oben wiedergegebenen Bedeutung substituiert sein kann. Damit bedeutet C₆- bis C₂₀-Aryl insbesondere auch Toluyl, Xylenyl, Pseudocumolyl oder Mesitylenyl.

Weiterhin soll im Zusammenhang mit der vorliegenden Erfindung unter C₇- bis C₂₀-Arylalkyl ein linear oder verzweigt C₁- bis C₅-Alkyl der oben wiedergegebene Bedeutung verstanden sein, der mit C₆- bis C₁₅-Aryl der oben wiedergegebenen Bedeutung substituiert ist. Insbesondere kann C₇- bis C₂₀-Arylalkyl einen Benzylrest bedeuten.

Weiterhin soll im Zusammenhang mit der vorliegenden Erfindung unter C₁- bis C₈-Alkyloxy ein Alkylrest mit 1 bis 8 Kohlenstoffatomen verstanden sein, der über ein Sauerstoffatom an ein Grundgerüst gebunden ist. Insbesondere kann C₁- bis C₈-Alkyloxy Methoxy, Ethoxy, n-Propyloxy, 1-Methylethyloxy (i-Propyloxy), n-Butyloxy, 1-Methylpropyoxyl, 2-Methylpropyloxy, n-Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy oder 1-Ethylpropyloxy bedeuten.

Weiterhin soll im Zusammenhang mit der vorliegenden Erfindung unter C₆- bis C₈-Aryloxy ein Arylrest mit 6 bis 8 Kohlenstoffatomen verstanden sein, der über ein Sauerstoffatom an ein Grundgerüst gebunden ist. Insbesondere kann C₆- bis C₈-Aryloxy Phenyloxy, Benzyloxy bedeuten.

Weiterhin soll im Zusammenhang mit der vorliegenden Erfindung unter C₁- bis C₈-Dialkylamino ein Aminorest verstanden sein, der mit zwei Alkylresten substituiert ist, die ihrerseits 1 bis 8 Kohlenstoffatome aufweisen. Insbesondere kann C₁- bis C₈-Dialkylamino Dimethylamino, Diethylamino oder Dipropylamino bedeuten. C₁- bis C₈-Dialkylamino kann somit auch als Di-C₁- bis C₈-alkylamino bezeichnet werden.

Da jeder der Reste R¹ = linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, weiterhin einfach oder mehrfach mit einem oder verschiedenen Substituenten ausgewählt aus der Gruppe Fluor, Chlor, C₁- bis C₈-Alkyloxy, C₆- bis C₈-Aryloxy, C₁- bis C₈-Dialkylamino, substituiert sein kann, kann selbstverständlich auch jeder hier einzeln aufgeführte Rest mit diesen Substituenten substituiert sein. So kann R¹ beispielsweise auch für 2-Methoxy-5-Chlorphenyl stehen.

Bevorzugt werden im Zusammenhang mit der vorliegenden Erfindung solche Reste R¹ = linear oder verzweigt C₁- bis C₂₀-Alkyl, C₃- bis C₂₀-Cycloalkyl, C₄- bis C₂₀-Cycloalkylalkyl, C₆-bis C₂₀-Aryl, C₇- bis C₂₀-Arylalkyl, die höchstens 15 Kohlenstoff-Atome, weiter bevorzugt höchstens 12 Kohlenstoffatome enthalten. Auch diese bevorzugten Reste R¹ können einfach oder mehrfach mit einem oder verschiedenen Substituenten ausgewählt aus der Gruppe Fluor, Chlor, C₁- bis C₈-Alkyloxy, C₆- bis C₈-Aryloxy, C₁- bis C₈-Dialkylamino, substituiert sein.

Somit bedeutet R¹ im Zusammenhang mit der vorliegenden Erfindung bevorzugt linear oder verzweigt C₁- bis C₁₅-Alkyl, nämlich ein linearer oder verzweigter Alkylrest mit 1 bis 15 Kohlenstoffatomen, der insbesondere die allgemeine Formel CₙH₂ₙ₊₁ aufweist, wobei n = eine ganze Zahl von 1 bis 15 bedeutet, und ganz bevorzugt linear oder verzweigt C₁- bis C₁₂-Alkyl, nämlich ein linearer oder verzweigter Alkylrest mit 1 bis 12 Kohlenstoffatomen, der insbesondere die allgemeine Formel CₙH₂ₙ₊₁ aufweist, wobei n = eine ganze Zahl von 1 bis 12 bedeutet.

Somit bedeutet R¹ im Zusammenhang mit der vorliegenden Erfindung bevorzugt C₃- bis C₁₅-Cycloalkyl, nämlich ein monocyclischer oder bicyclischer Cycloalkylrest mit 3 bis 15 Kohlenstoffatomen, wobei n = eine ganze Zahl von 3 bis 15 bedeutet, und besonders bevorzugt C₃- bis C₁₂-Cycloalkyl, nämlich ein monocyclischer oder bicyclischer Cycloalkylrest mit 3 bis 12 Kohlenstoffatomen, wobei n = eine ganze Zahl von 3 bis 12 bedeutet.

Somit bedeutet R¹ im Zusammenhang mit der vorliegenden Erfindung bevorzugt C₄- bis C₁₅-Cycloalkylalkyl, wobei hierunter ein linear oder verzweigt C₁- bis C₅-Alkyl der oben angegebenen Bedeutung verstanden sein soll, der seinerseits mit C₃- bis C₁₀-Cycloalkyl substituiert ist, und der insbesondere die allgemeine Formel CₙH₂ₙ₋₁ aufweist, wobei n = eine ganze Zahl von 4 bis 15 bedeutet. Ganz besonders bevorzugt bedeutet R¹
C₄- bis C₁₂-Cycloalkylalkyl, wobei hierunter ein linear oder verzweigt C₁- bis C₅-Alkyl der oben angegebenen Bedeutung verstanden sein soll, der seinerseits mit C₃- bis C₁₀-Cycloalkyl substituiert ist, und der insbesondere die allgemeine Formel CₙH₂ₙ₋₁ aufweist, wobei n = eine ganze Zahl von 4 bis 12 bedeutet.

Somit bedeutet R¹ im Zusammenhang mit der vorliegenden Erfindung bevorzugt
C₆- bis C₁₅-Aryl, nämlich ein aromatischer Arylrest mit 6 bis 15 Kohlenstoffatomen, insbesondere ein monocyclischer aromatischer Arylrest mit 6 bis 15 Kohlenstoffatomen oder ein bicyclischer aromatischer Arylrest mit 6 bis 15 Kohlenstoffatomen oder ein tricyclischer aromatischer Arylrest mit 6 bis 15 Kohlenstoffatomen, der weiterhin bevorzugt seinerseits einfach oder mehrfach mit linear oder verzweigt C₁- bis C₅-Alkyl der oben wiedergegebenen Bedeutung substituiert sein kann. Besonders bevorzugt bedeutet R¹ C₆- bis C₁₂-Aryl, nämlich ein aromatischer Arylrest mit 6 bis 12 Kohlenstoffatomen, insbesondere ein monocyclischer aromatischer Arylrest mit 6 bis 12 Kohlenstoffatomen oder ein bicyclischer aromatischer Arylrest mit 6 bis 12 Kohlenstoffatomen, der weiterhin bevorzugt seinerseits einfach oder mehrfach mit linear oder verzweigt C₁- bis C₅-Alkyl der oben wiedergegebenen Bedeutung substituiert sein kann.

Somit bedeutet R¹ im Zusammenhang mit der vorliegenden Erfindung bevorzugt C₇- bis C₁₅-Arylalkyl, wobei hierunter ein linear oder verzweigt C₁- bis C₅-Alkyl der oben wiedergegebenen Bedeutung verstanden sein soll, der mit C₆- bis C₁₀-Aryl substituiert ist. Besonders bevorzugt bedeutet R¹ C₇- bis C₁₂-Arylalkyl, wobei hierunter ein linear oder verzweigt C₁- bis C₅-Alkyl der oben wiedergegebenen Bedeutung verstanden sein soll, der mit C₆- bis C₈-Aryl substituiert ist.

Die in dem erfindungsgemäßen Verfahren eingesetzte magnesiumorganische Verbindung der Formel (II) wird in bekannter Weise aus einem entsprechenden Alkylhalogenid R¹-X mit X = Chlor, Brom, lod, vorzugsweise X = Chlor, und elementarem Magnesium in einem organischen Lösungsmittel unter Bedingungen, die für Grignard-Reaktionen üblich sind, hergestellt.

Das in dem erfindungsgemäßen Verfahren eingesetzte *N*,*N*'-Bis(trialkylsilyl)carbodiimid gemäß Formel (III) kann z.B. gemäß der deutschen Patentschrift DE 198 22 281 C1 aus wasserfreiem Cyanamid und Hexaalkyldisilazan hergestellt werden. So lässt sich *N*,*N'-*Bis(trimethylsilyl)carbodiimid (nachfolgend auch BTSCD) aus wasserfreiem Cyanamid und Hexamethyldisilazan herstellen und durch Destillation in reiner Form gewinnen. Analog sind andere *N*,*N'*Bis(trialkylsilyl)carbodiimide gemäß Formel (III) mit R² = gleichzeitig oder unabhängig voneinander linear oder verzweigt C₁- bis C₅-Alkyl, herzustellen, und im erfindungsgemäßen Verfahren einzusetzen. Bevorzugt ist R² unabhängig bei jedem Auftreten Methyl oder Ethyl, besonders bevorzugt jeweils Methyl.

Die Umsetzung der magnesiumorganischen Verbindung gemäß Formel (II) mit *N*,*N*'-Bis(trialkylsilyl)carbodiimid gemäß Formel (III), insbesondere BTSCD, in einem organischen Lösungsmittel erfolgt vorzugsweise durch Zugabe des *N*,*N*'-Bis(trialkylsilyl)carbodiimid gemäß Formel (III), insbesondere BTSCD, zu einer Lösung, insbesondere einer etherischen Lösung, der magnesiumorganischen Verbindung R¹-Mg-X. Alternativ kann auch die magnesiumorganischen Verbindung R¹-Mg-X zu dem *N*,*N'*Bis(trialkylsilyl)carbodiimid, insbesondere BTSCD, das in einem geeigneten organischen Lösungsmittel gelöst ist, dosiert werden. Geeignete Lösemittel oder Lösungsmittel sind die üblicherweise für Grignard-Reaktionen verwendeten Lösungsmittel.

Besonders bevorzugt können in dem erfindungsgemäßen Verfahren als organisches Lösungsmittel eingesetzt werden:
i) ein Ether, ausgewählt aus der Gruppe Diethylether, Di-n-butylether, t-Butyl-methylether, Dimethoxymethan, Diethoxymethan, 1,2-Dimethoxymethan, Tetrahydrofuran, 1,4-Dioxan, sowie Gemische hiervon, oder
ii) ein Kohlenwasserstoff, ausgewählt aus der Gruppe Petroleumfraktionen, n-Pentan, n-Hexan, n-Heptan, n-Octan, Isooctan, Cyclohexan, Methylcyclohexan, Benzol, Toluol oder Xylol, sowie Gemische hiervon, oder
iii) ein Gemisch aus i) und ii).

Gemäß einer besonders bevorzugten Ausführung der vorliegenden Erfindung kann dabei vorgesehen sein, dass für den Rest R¹ gilt:
R¹ = linear oder verzweigt C₁- bis C₂₀-Alkyl oder C₃- bis C₂₀-Cycloalkyl.

Gemäß einer ganz besonders bevorzugten Ausführung der vorliegenden Erfindung kann dabei vorgesehen sein, dass für den Rest R¹ gilt:
R¹ = linear oder verzweigt C₁- bis C₁₅-Alkyl oder C₃- bis C₁₅-Cycloalkyl.

Gleichzeitig oder unabhängig hiervon kann als *N*,*N*'-Bis(trialkylsilyl)carbodiimid gemäß Formel (III) insbesondere BTSCD eingesetzt werden.

Besonders bevorzugt kann die Umsatzung der magnesiumorganischen Verbindung gemäß Formel (II) mit dem *N*,*N*'-Bis-trialkylsilyl-carbodiimide gemäß Formel (III), insbesondere mit BTSCD, in Stufe a) bei einer Temperatur im Bereich von - 40 °C bis 180 °C, insbesondere im Bereich von -20°C bis 100°C und ganz besonders bevorzugt im Bereich 0°C bis 50°C, und/ oder bei einem Druck im Bereich von 0,01 bis 10.000 mbar, insbesondere im Bereich von 20 bis 2.000 mbar, durchgeführt werden.

Weiterhin bevorzugt kann dabei gleichzeitig oder unabhängig von den eingestellten Temperatur- oder Druckbereichen vorgesehen sein, dass in Verfahrensstufe a) pro Mol magnesiumorganische Verbindung gemäß Formel (II) 0,8 bis 1,2 Mol des *N*,*N*'-Bis(trialkylsilyl)carbodiimid gemäß Formel (lll), insbesondere BTSCD, eingesetzt wird.

Das bei der Umsetzung erhaltene Magnesium-Carboxamidinat gemäß Formel (IV) wird anschließend hydrolysiert, insbesondere mittels Wasser und/oder Säure und/oder einem C₁- bis C₅-Alkohol hydrolysiert. Besonders bevorzugt ist dabei vorgesehen, dass in Verfahrensstufe b) als Säure Salzsäure und/oder ein C₁- bis C₅-Alkohol, insbesondere Ethanol oder Isopropanol eingesetzt wird.

Vorzugsweise wird aus der Reaktionsmischnung das in Verfahrensschritt a) verwendete organische Lösungsmittel, insbesondere der Ether und/ oder der Kohlenwasserstoff und/ oder ein Gemisch hiervon, destillativ abgetrennt und recycelt. Das erhaltene trockene Salzgemisch wird dann in vorgelegtes Wasser eingetragen, mit einer Säure, vorzugsweise Salzsäure, angesäuert und bis zur vollständigen Hydrolyse der Silylgruppen gerührt. Es resultiert ein Gemisch aus dem gewünschten Carboxamidin-Hydrochlorid, Magnesiumchlorid, und Trimethylsilanol bzw. dessen Kondensationsprodukt Hexamethyldisiloxan (die Silane bilden eine eigene, organische Phase). Durch simples Eindampfen werden die Silane abdestilliert und das restliche Wasser soweit als möglich herausgetrocknet. Es verbleibt ein Gemisch aus Magnesiumchlorid-Hexahydrat und dem gewünschten Carboxamidin-Hydrochlorid.

Weiterhin bevorzugt ist auch ein Verfahren Gegenstand der vorliegenden Erfindung in dem in Verfahrensstufe b) das Hydrolysierungsmittel in einem 1- bis 10-fachen molaren Überschuss, bezogen auf die eingesetzte molare Menge der magnesiumorganischen Verbindung der allgemeinen Formel (II), einsetzt wird.

Gleichzeitig oder unabhängig von der eingesetzten Menge an Hydrolysierungsmittel kann in dem erfindungsgemäßen Verfahren in Verfahrensstufe b) als Hydrolysierungsmittel Salzsäure eingesetzt werden und/oder vorgesehen sein, dass das *N*,*N*'-unsubstituierte Carboxamidin gemäß Formel (I) als Hydrochlorid isoliert wird.

Die freie Base der Carboxamidine kann z.B. durch Umsetzung eines Carboxamidin-Hydrochlorids bzw. eines Gemisches desselben mit einem Magnesiumhalogenid mit einem Alkoholat, bevorzugt einem Alkalimetall-Alkoholat, insbesondere einem Alkalimetall-Alkoholat eines C₁ bis C₄-Alkohols und eines Alkalimetalls aus der Gruppe Lithium, Kalium, Natrium, und Filtration der ausgefallenen Salze erhalten werden.

Ganz besonders bevorzugt kann die Isolierung in Verfahrensstufe c) durch Zugabe eines organischen, nicht wasserlöslichen Lösungsmittels mittels Extraktion und anschließendes Verdampfen des nicht wasserlöslichen Lösungsmittels erfolgen.

Somit wird gemäß einer weiterführenden Ausführung auch die Verwendung von *N*,*N'*-Bis(alkylsilyl)carbodiimiden gemäß Formel (III) zur Herstellung von *N,N'*-unsubstituierten Carboxamidinen, insbesondere der allgemeinen Formel (I) R¹-C(=NH)-NH₂, beschrieben, wobei für Formel (III) gilt

(R²)₃Si-N=C=N-Si(R²)₃ **Formel (III)**

wobei für die Reste R² gleichzeitig oder unabhängig voneinander gilt,
- R² =: gleichzeitig oder unabhängig voneinander linear oder verzweigt C₁- bis C₅-Alkyl, insbesondere gleichzeitig oder unabhängig voneinander C₁- bis C₂-Alkyl.

Ganz besonderes bevorzugt kann dabei als *N,N*'-Bis(alkylsilyl)carbodiimiden *N*,*N'*-Bis(methylsilyl)carbodiimid verwendet werden.

Weitergehende Untersuchungen haben gezeigt, dass die in Verfahrensstufe a) intermediär gebildeten, silylierten Magnesium-Carboxamidinate gemäß Formel (IV) auch direkt zur Synthese von Heterocyclen eingesetzt werden können. Somit wird gemäß einem weiterführenden Gedanken auch die Verwendung von silylierten Magnesium-Carboxamidinaten gemäß Formel (IV) zur Herstellung von stickstoffhaltigen Heterocyclen, insbesondere zur Herstellung von Pyrimidinen und Triazinen, beschrieben, wobei für Formel (IV) gilt

R¹-C(=N-Si(R²)₃)-N(-Si(R²)₃)·Mg-X **Formel (IV)**

wobei für die Reste R¹, R² und X gleichzeitig oder unabhängig gilt:
- R¹ =: linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino,
- R² =: gleichzeitig oder unabhängig voneinander linear oder verzweigt C₁- bis C₅-Alkyl,
- X =: Chlor, Brom oder lod.

Weiterhin wird auch ein Verfahren zur Herstellung von Pyrimidinen oder Triazinen gemäß der allgemeinen Formel (V) beschrieben, wobei für Formel (V) gilt: 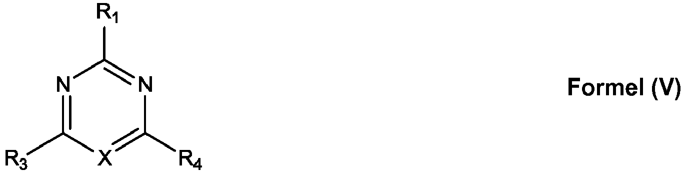 wobei für die Rest R¹, R³, R⁴ unabhängig voneinander gilt:
- R¹ =: linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste R¹ weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino,
- R³, R⁴ =: unabhängig voneinander OH, NH₂, NHR⁵, NR⁵R⁶, C₁- bis C₂₀-Alkyl, C₆- bis C₂₀-Aryl,
- R⁵, R⁶ =: unabhängig voneinander C₁- bis C₂₀-Alkyl,
- X=: CH oder N,
umfassend die Verfahrensschritte:
A1) Herstellen eines *N,N'*-unsubstituierten Carboxamidin der allgemeinen Formel (I) oder eines Salzes hiervon gemäß der vorliegenden Erfindung, insbesondere Herstellen eines *N,N'*-unsubstituierten Carboxamidin der allgemeinen Formel (I) oder eines Salzes hiervon, wobei für die Formel (I) gilt:

   R¹-C(=NH)-NH₂ **Formel (I)**

   wobei für den Rest R¹ gilt:
   - R¹ =: linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste R¹ weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino,
   wobei das *N,N'*-unsubstituierten Carboxamidin durch Verfahrensschritte a), b) und c) hergestellt wird:
   a) Umsetzung einer magnesiumorganischen Verbindung der allgemeinen Formel (II) mit einem *N,N*'Bis(trialkylsilyl)carbodiimid gemäß Formel (III) in einem organischen Lösungsmittel, wobei für Formel (II) und Formel (III) gilt:

      R¹-Mg-X **Formel (II)**

      wobei für den Rest R¹ die oben genannte Bedeutung und für den Rest X gilt:
      X = Chlor, Brom oder lod,

      (R²)₃Si-N=C=N-Si(R²)₃ **Formel (III)**

      wobei für die Reste R² gleichzeitig oder unabhängig voneinander gilt,
      - R² =: gleichzeitig oder unabhängig voneinander linear oder verzweigt C₁- bis C₅-Alkyl,
   b) Hydrolysieren des in Schritt a) intermediär gebildeten, silylierten Magnesium-Carboxamidinat gemäß Formel (IV)

      R¹-C(=N-Si(R²)₃)-N(-Si(R²)₃) Mg-X **Formel (IV)**

      wobei für die Reste R¹, R² und X die oben genannte Bedeutung gilt,
   c) Isolieren der *N,N*'unsubstituierten Carboxamidine der allgemeinen Formel (I) oder eines Salzes hiervon,
      oder
A2) Herstellen eines *N,N'*-unsubstituierten Carboxamidin der allgemeinen Formel (I) oder
   eines Salzes hiervon, wobei das *N,N'*-unsubstituierten Carboxamidin durch die Verfahrensschritte a) und b) hergestellt wird:
   a) Umsetzung einer magnesiumorganischen Verbindung der allgemeinen Formel (II) mit einem *N*,*N*'-Bis(trialkylsilyl)carbodiimid gemäß Formel (III) in einem organischen Lösungsmittel, wobei für Formel (II) und Formel (III) gilt:

      R¹-Mg-X **Formel (II)**

      wobei für den Rest R¹ die oben genannte Bedeutung und für den Rest X gilt:
      X = Chlor, Brom oder lod,

      (R²)₃Si-N=C=N-Si(R²)₃ **Formel (III)**

      wobei für die Reste R² gleichzeitig oder unabhängig voneinander gilt,
      - R² =: gleichzeitig oder unabhängig voneinander linear oder verzweigt C₁- bis C₅-Alkyl,
   b) Hydrolysieren des in Schritt a) intermediär gebildeten, silylierten Magnesium-Carboxamidinat gemäß Formel (IV)

      R¹-C(=N-Si(R²)₃)-N(-Si(R²)₃)·Mg-X **Formel (IV)**

      wobei für die Reste R¹, R² und X die oben genannte Bedeutung gilt,
      oder
A3) Herstellen eines silylierten Magnesium-Carboxamidinate der allgemeinen Formel (IV) gemäß Verfahrensschritt a)
   a) Umsetzung einer magnesiumorganischen Verbindung der allgemeinen Formel (II) mit einem *N*,*N*'-Bis(trialkylsilyl)carbodiimid gemäß Formel (III) in einem organischen Lösungsmittel, wobei für Formel (II) und Formel (III) gilt:

      R¹-Mg-X **Formel (II)**

      wobei für den Rest R¹ die oben genannte Bedeutung und für den Rest X gilt:
      X = Chlor, Brom oder lod,

      (R²)₃Si-N=C=N-Si(R²)₃ **Formel (III)**

      wobei für die Reste R² gleichzeitig oder unabhängig voneinander gilt,
      - R² =: gleichzeitig oder unabhängig voneinander linear oder verzweigt C₁- bis C₅-Alkyl,
   und
B) Umsetzung des in Verfahrensschritt A1), A2) oder A3) hergestellten Produktes mit einer Verbindung der allgemeinen Formel (VI), wobei für Formel (VI) gilt: 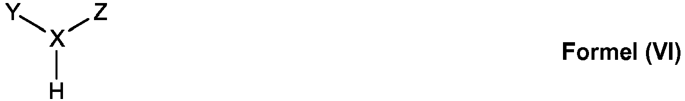 wobei für die Reste X, Y und Z gilt:
   X = CH, N,
   Y, Z = unabhängig voneinander CN oder ein Rest der folgenden Formeln (VII), (VIII) oder (IX):

   R⁶-C(O)- **Formel (VII)**

   wobei für den Rest R⁶ gilt:
   R⁶ = C₁- bis C₂₀-Alkyl, C₆- bis C₂₀-Aryl, C₇- bis C₂₀-Arylalkyl,

   R⁷-O-C(O)- **Formel (VIII)**

   wobei für den Rest R⁶ gilt:
   R⁷ = C₁- bis C₂₀-Alkyl,

   R⁸R⁹N-C(O)- **Formel (IX)**

   wobei für die Reste R⁸ und R⁹ unabhängig voneinander gilt:
   R⁸, R⁹ = Wasserstoff, C₁- bis C₂₀-Alkyl, C₆- bis C₂₀-Aryl, C₇- bis C₂₀-Arylalkyl.

Als Verbindungen der Formel (VI) können hierbei besonders bevorzugt 1,3-Diketone, Acylessigester, Cyanessigester, Malonsäurenitrile, Alkoxycarbonylcyanamide und N-Cyancarbonimidate eingesetzt werden. Die Umsetzung im Verfahrensschritt B) kann dabei unter für Pyrimidinsynthesen und Triazinsynthesen üblichen Reaktionsbedingungen erfolgen. Im Verfahrensschritt B) haben sich Alkohole ggf. unter Zusatz von Alkalimetallalkoholaten als vorteilhaft erwiesen.

Bevorzugte Ausführungen dieses Verfahrens entsprechen den bevorzugten Ausführungen des hierin beschriebenen Verfahrens zur Herstellung von *N,N*'-unsubstituierten Carboxamidin der allgemeinen Formel (I) oder eines Salzes hiervon.

Nachfolgend soll die Erfindung anhand von Beispielen näher erläutert werden.

### Beispiele:

### 1.) Herstellung von Cyclopentan-carboxamidin-Hydrochlorid

12,2 g (0,5 mol) Magnesiumspäne wurden in 160 g Diethylether mit 53,3 g (0,51 mol) Cyclopentylchlorid zu einer Lösung von Cyclopentylmagnesiumchlorid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Es wurden 149,2 g Feststoff erhalten.

Der Feststoff zeigte folgende NMR-Daten: ¹H-NMR in DMSO: 1,93 ppm (m, 1H), 1,66 ppm (m, 2H), 1,54 ppm (m, 2H), -0,03 ppm (s, 18 H), ¹³C-NMR: 124,1 ppm (Amidinat-C), 39,6 ppm (CH), 30,59 ppm (CH₂), 24,65 ppm (CH₂), 2,68 ppm (SiCH₃). Damit wurde die Struktur des Amidinats gemäß Formel IV bestätigt.

Der erhaltene Feststoff wurde in 325 g Eiswasser eingetragen, mit 135,0 g 25%iger Salzsäure auf einen pH 1,0 gestellt und 3 Stunden bei 40°C bis zur Vervollständigung der Hydrolyse gerührt. Das erhaltene 2-phasige Gemisch (Magnesiumchlorid und Amidin-Hydrochlorid in der Wasserphase, Silanol bzw. Siloxan und Kohlenwasserstoffe, z.B. Cyclopentan in der organischen Phase) wurde einrotiert, wobei die organische Phase vollständig in das Destillat überging. Nach Nachtrocknung verblieben 159,1 g eines leicht klebrigen Feststoffs. Dieser wurde per NMR analysiert, es wurde auschließlich das gewünschte Carboxamidin in reiner Form detektiert (Magnesiumchlorid wurde nicht detektiert). ¹H-NMR in DMSO: 9,18 ppm, 8,95 ppm (zwei NH2-Gruppen, je 2 H), 2,84 ppm (quintett, 1H), 1,90 ppm (m, 2H), 1,71 ppm (m, 4H), 1,51 ppm (m, 2H). ¹³C-NMR: 174,46 ppm (Amidin-C), 42,15 ppm (CH), 30,59 ppm, 25,21 ppm (2 Paare CH₂). Weitere Signale (außer H₂O und DMSO) konnten im NMR nicht detektiert werden. Mittels LC/MS wurde die Masse des Aminidiumions (M=113) bestätigt. Der Gehalt an Cyclopentancarboxamidin-Hydrochlorid betrug 43%, die erhaltene Ausbeute war quantitativ.

### 2.) Herstellung von Isobutanamidin-Hydrochlorid

12,2 g (0,5 mol) Magnesiumspäne wurden in 160 g Diethylether mit 40,1 g (0,51 mol) Isopropylchlorid zu einer Lösung von Isopropylmagnesiumchlorid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Der erhaltene Feststoff (148,7 g) wurde in 325 g Eiswasser eingetragen, mit 133,9 g 25%iger Salzsäure auf einen pH 1,0 gestellt und 3 Stunden bei 40°C bis zur Vervollständigung der Hydrolyse gerührt. Das erhaltene 2-phasige Gemisch (Magnesiumchlorid und Amidin-Hydrochlorid in der Wasserphase, Silanol bzw. Siloxan und Kohlenwasserstoffe, z.B. Cyclopentan in der organischen Phase) wurde einrotiert, wobei die organische Phase vollständig in das Destillat überging. Nach Nachtrocknung verblieben 159,2 g eines leicht klebrigen Feststoffs. Dieser wurde per NMR analysiert, es wurde auschließlich das gewünschte Carboxamidin in reiner Form detektiert (Magnesiumchlorid wurde nicht detektiert). ¹H-NMR in DMSO: 9,15 ppm, 8,88 ppm (je 2 Amidin-NH), 2,49 ppm (septett 1H), 1,15 ppm (d, 6H). ¹³C-NMR: 175,8 ppm (Amidin-C), 31,95 ppm (CH), 19,26 ppm (2 CH₃). Weitere Signale konnten im NMR nicht detektiert werden. Mittels LC/MS wurde die Masse des Amidiniumions (M=87) bestätigt. Der Gehalt an Isobutanamidin-Hydrochlorid betrug 32%, die erhaltene Ausbeute bezogen auf die eingesetzte Menge BTSCD betrug somit 92%.

### 3.) Vergleichsbeispiel: Umsetzung mit nicht-silyliertem Cyanamid

12,2 g Magnesiumspäne wurden in 160 g Diethylether mit 53,3 g Cyclopentylchlorid umgesetzt. Dazu wurde eine Lösung von 6,3 g (0,15 mol) Cyanamid F1000 in 50 g Diethylether dosiert, wobei ein weisser Feststoff ausfiel. Nach Eindampfen und analoger Aufarbeitung wie in Beispiel 1 wurden 124 g eines klebrigen Feststoffs erhalten. Darin konnte keinerlei Amidin nachgewiesen werden. Lediglich Dicyandiamid als Reaktionsprodukt von Cyanamid im alkalischen Medium wurde detektiert. Hierdurch wird gezeigt, dass auch durch einen großen Überschuß an Grignard-Reagens unsubstituiertes Cyanamid nicht in gewünschter Form am C-Atom reagiert, sondern schlicht als Protonensäure mit dem Grignard-Reagens unter Bildung des Kohlenwasserstoffs R-H abreagiert. Die Silylierung von Cyanamid zu BTSCD ist damit essentiell für die Bildung von Amidinen.

### 4.) Herstellung von n-Nonanamidin-Hydrochlorid

12,2 g (0,5 mol) Magnesiumspäne wurden in 160 g Diethylether mit 75,8 g (0,51 mol) 1-Chloroctan zu einer Lösung von n-Octylmagnesiumchlorid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Dabei wurden 170,8 g eines halbfesten Rückstandes erhalten. Dieser wurde in 325 g Eiswasser eingetragen, mit 117 g 32%iger Salzsäure auf einen pH 1,0 gestellt und 3 Stunden bei 40°C bis zur Vervollständigung der Hydrolyse gerührt. Das erhaltene 2-phasige Gemisch aus Magnesiumchlorid, Amidin-Hydrochlorid und Silanol bzw. Siloxan wurde einrotiert. Es blieb wiederum eine zweiphasige Mischung, wobei die obere, organische Flüssigphase als Produkt abgetrennt wurde. Nach Nachtrocknung verblieben 115,6 g einer halbfesten Masse. ¹H-NMR-Daten in DMSO: 9,05 ppm (breit, 4H), 2,38 ppm (t, 2H), 1,58 ppm (tt, 2H), 1,23 ppm (m, 10H), 0,83 (t, 3H). ¹³C-NMR: 171,3 ppm (Amidin-C), 31,53 ppm, 31,24 ppm, 28,53 ppm, 28,49 ppm, 28,21 ppm, 26,36 ppm, 22,09 ppm (7 CH₂-Gruppen), 13,98 ppm (CH₃). Weitere Signale (außer Wasser und DMSO) konnten im NMR nicht detektiert werden. Im LC/MS wurde die Masse des Amidinium-Kations (157) bestätigt. Der Gehalt an Nonanamidin-Hydrochlorid betrug 74%, die isolierte Ausbeute somit 99%.

### 5.) Herstellung von 3-Ethyl-heptanamidin-Hydrochlorid

12,2 g (0,5 mol) Magnesiumspäne wurden in 160 g Diethylether mit 75,8 g (0,51 mol) 1-Chlor-2-ethyl-hexan zu einer Grignard-Lösung umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Dabei wurden 168,5 g eines halbfesten Rückstandes erhalten. Dieser wurde in 325 g Eiswasser eingetragen, mit 121,5 g 32%iger Salzsäure auf einen pH 1,0 gestellt und 3 Stunden bei 40°C gerührt. Das erhaltene 2-phasige Gemisch einrotiert. Es blieb wiederum eine zweiphasige Mischung, wobei die obere, organische Flüssigphase als Produkt abgetrennt und mit Wasser gewaschen wurde. Nach Nachtrocknung verblieben 93,1 g einer hochviskosen Flüssigkeit. ¹H-NMR-Daten in DMSO: 9,03 ppm, 8,78 ppm (Amidin-NH, je 2H), 2,28 ppm (m, 2H), 1,80 ppm (m, 1H), 1,31 - 1,16 ppm (breites Multiplett, 8H), 0,86, 0,83 ppm (je Triplett, überlagert, 2 x 3H). ¹³C-NMR: 170,73 ppm (Amidin-C), 36,49 ppm, 36,36 ppm, 31,51 ppm, 27,68 ppm, 24,84 ppm, 22,35 ppm, 13,89 ppm, 10,07 ppm. Weitere Signale (außer Wasser und DMSO) konnten im NMR nicht detektiert werden. Im LC/MS wurde die Masse des Kations (157) bestätigt. Der Gehalt an 3-Ethylheptanamidin-Hydrochlorid betrug 90%, die isolierte Ausbeute somit 97%.

### 6.) Herstellung von Pivalinamidin-Hydrochlorid (2,2-Dimethylpropanamidin-Hydrochlorid)

12,2 g (0,5 mol) Magnesiumspäne wurden in 160 g Diethylether mit 47,2 g (0,51 mol) tert-Butylchlorid zu einer Grignard-Lösung umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Dabei wurden 110,5 g eines weissen Feststoffs erhalten. Dieser wurde in 300 g Eiswasser eingetragen, mit 121 g 32%iger Salzsäure auf einen pH 1,0 gestellt und 3 Stunden bei 40°C gerührt. Das erhaltene 2-phasige Gemisch einrotiert. Es blieb gelbliche, klare Lösung. Beim Abkühlen auf -5°C kristallisierten 14,6 g eines farblosen Feststoffs, der abfiltriert und getrocknet wurde. ¹H-NMR-Daten in DMSO: 9,12 ppm, 8,78 ppm (Amidin-NH, je 2H), 1,21 ppm (s, 9H). ¹³C-NMR: 171,7 ppm (Amidin-C), 36,38 ppm (C2), 27,06 ppm (3 CH₃). Weitere Signale (außer Wasser und DMSO) konnten im NMR nicht detektiert werden. Die Massen des Amidinium-lons (101) konnte per LC/MS bestätigt werden. Das isolierte Produkt hatte einen Gehalt von 80%, die isolierte Ausbeute betrug somit 19%.

Das Filtrat aus obiger Synthese wurde zur Trockene eingedampft und weitere 138,8 g eines Produkt/Salz-Gemisches erhalten. Im NMR konnten nur die Signale des Zielprodukts detektiert werden, der Gehalt an Pivalinamidin-Hydrochlorid betrug 25%, was zusätzlichen 57% Ausbeute entspricht. Die Gesamtausbeute betrug somit 76%.

### 7.) Herstellung von 4-Methyl-benzamidin-Hydrochlorid

12,2 g (0,5 mol) Magnesiumspäne wurden in 160 g Diethylether mit 87,2 g (0,51 mol) 4-Bromtoluol zu einer Lösung von 4-Methylphenyl-magnesiumbromid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die gelbe Suspension des Magnesium-Amidinats wurde zur Trockene eingedampft. Dabei wurden 198,9 g eines weissen Feststoffs erhalten. Dieser wurde in 500 g Eiswasser eingetragen, mit 110 g 32%iger Salzsäure auf einen pH 1,0 gestellt und 3 Stunden bei 40°C gerührt. Die erhaltene Suspension wurde am Rotationsverdampfer bei 45°C/ 30 mbar von entstandenen Silanen befreit und dann auf 20°C abgekühlt. Der ausgefallene Feststoff wurde abfiltriert, mit Wasser gewaschen und bei 60°C getrocknet. Es wurden 112,6 g eines weissen Feststoffs erhalten. ¹H-NMR-Daten in DMSO: 9,40, 9,20 ppm, (Amidin-NH, 2 x 2H), 7,78 ppm (d, 2H), 7,40 ppm (d, 2H), 2,39 ppm (s, 3H). ¹³C-NMR: 165,5 ppm (Amidin-C), 144,5, 129,5, 128,1, 124,9, 21,1 ppm. Per NMR wurden ca. 2 mol% 4,4'-Dimethylbiphenyl gefunden. Weitere Signale (außer Wasser und DMSO) konnten nicht detektiert werden. Der Gehalt an 4-Methylbenzamidinhyddrochlorid betrug 60%, die Ausbeute somit 88%.

### 8.) Herstellung von 2-Methoxy-5-chlor-benzamidin-Hydrochlorid

12,2 g (0,5 mol) Magnesiumspäne wurden in 160 g Diethylether mit 113,0 g (0,51 mol) 2-Brom-4-chloranisol zu einer Lösung von 5-Chlor-2-methoxyphenyl-magnesiumbromid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die gelbe Suspension des Magnesium-Amidinats wurde zur Trockene eingedampft. Dabei wurden 241,4 g eines beigen Feststoffs erhalten. Dieser wurde in 500 g Eiswasser eingetragen, mit 122 g 32%iger Salzsäure auf einen pH 1,0 gestellt und 3 Stunden bei 40°C gerührt. Die erhaltene Suspension wurde abfiltriert, mit Wasser gewaschen und bei 60°C getrocknet. Es wurden 101,2 g eines gelblichen Feststoffs erhalten. ¹H-NMR-Daten in DMSO: 9,24 ppm, (Amidin-NH, 4H), 7,66 ppm (m, 2H), 7,27 ppm (d, 1H), 3,85 ppm (s, 3H, OCH₃). ¹³C-NMR: 163,1 ppm (Amidin-C), 155,58, 133,29, 129,05, 124,06, 119,60, 114,33 ppm (6 Aromaten-C), 56,59 ppm (OCH₃). Weitere Signale (außer Wasser und DMSO) konnten nicht detektiert werden. Der Gehalt an 2-Methoxy-5-chlor-benzamidin-Hydrochlorid betrug 82%, die isolierte Ausbeute somit 83%.

### 9.) Direkte weitere Umsetzung eines Amidin-Hydrochlorids zu einem 1,3,5-Triazin:

### Herstellung von 2-Amino-4-methyl-6-octyl-1,3,5-triazin

12,2 g (0,5 mol) Magnesiumspäne wurden in 160 g Diethylether mit 75,8 g (0,51 mol) 1-Chloroctan zu einer Lösung von n-Octylmagnesiumchlorid umgesetzt. Zu dieser wurden bei 35°C über 2 Stunden 83,9 g (0,45 mol) Bis-trimethylsilylcarbodiimid dosiert und die Mischung über Nacht bei 35°C nachreagiert. Die weisse Suspension des Magnesium-Amidinats wurde zur Trockene eingedampft. Der dabei erhaltene halbfeste Rückstand wurde in 400 g Eiswasser eingetragen. Zu der erhaltenen Suspension wurden bei 0°C 44,2 g (0,45 mol) Methyl-N-cyanacetimidat zugetropft und nach Zugabeende für 2 Stunden bei 40°C nachreagiert.

Die weisse, dicke Suspension wurde mit 79,0 g 32 %-iger Salzsäure auf pH 4 gestellt, 1 Stunde bei 40°C gerührt und dann mit 3,6 g 25 %-iger Natronlauge auf pH 6 gestellt. Das halbfeste Produkt schied sich am Boden des Gefäßes ab und kristallisierte über mehrere Stunden. Die Wasserphase wurde dekantiert, der Rückstand mit Wasser gewaschen und bei 60°C im Vakuum getrocknet. Es wurden 101,6 g Produkt erhalten.

Umkristallisation aus 100 g Ethanol ergab 59,7 g reines 2-Amino-4-methyl-6-octyl-1,3,5-triazin mit Schmelzpunkt 68,8 bis 72,3°C. Die Ausbeute betrug 60%. ¹H-NMR-Daten in CDCl₃: 8,56, 8,34 ppm, (NH₂, 2H), 2,50 ppm (t, 2H), 2,21 ppm (s, 3H), 1,72 ppm (m, 2H), 1,37 ppm (m, 2H), 1,29-1,24 ppm (m, 8H), 0,86 (t, 3H). ¹³C-NMR: 181,2, 170,9, 170,8 ppm (Triazin-C), 31,87, 31,22, 28,49, 28,43, 26,30, 22,75, 22,09, 13,97 ppm. Per GC wurde ein Gehalt von 97,8% bestimmt. Das Massenspektrum ergab folgende Fragmentierung: 222 (M+), 221, 193, 179, 165, 151, 137, 124, 43 amu.

### 10.) Herstellung von Nonanamidin (freie Base)

85 g des halbfesten Produktgemisches aus Beispiel 4, enthaltend 65,5 g n-Nonanamidin-Hydrochlorid und restliches Magnesiumchlorid wurden in 200 g Ethanol gelöst. Hierzu wurden bei 0°C 264,2 g einer 21%igen Lösung von Natriumethylat in Ethanol getropft. Ausgefallene Salze wurden abfiltriert und mit Ethanol gewaschen, die vereinigten Filtrate im Vakuum zur Trockene eingedampft. Es wurden 48,4 g einer wachsartigen, halbfesten Masse erhalten. Die Elementaranalyse (17,8% N, 69,1% C) bestätigte die Struktur des erhaltenen Nonanamidins (freie Base).

## Patentansprüche

1. Verfahren zur Herstellung von *N,N'*-unsubstituierten Carboxamidinen der allgemeinen Formel (I) oder einem Salz hiervon
R¹-C(=NH)-NH₂ **Formel (I)**
wobei für den Rest R¹ gilt:
R¹ = linear oder verzweigt C₁- bis C₂₀-Alkyl, C₃- bis C₂₀-Cycloalkyl, C₄- bis C₂₀-Cycloalkylalkyl, C₆- bis C₂₀-Aryl, C₇- bis C₂₀-Arylalkyl, wobei diese Reste weiterhin einfach oder mehrfach substituiert sein können und die Substituenten ausgewählt werden aus der Gruppe Fluor, Chlor, C₁- bis C₈-Alkyloxy, C₆- bis C₈-Aryloxy, C₁- bis C₈-Dialkylamino,
umfassend die Verfahrensschritte
a) Umsetzung einer magnesiumorganischen Verbindung der allgemeinen Formel (II) mit einem *N*,*N*'-Bis(trialkylsilyl)carbodiimid gemäß Formel (III) in einem organischen Lösungsmittel, wobei für die Formel (II) und Formel (III) gilt:
R¹-Mg-X **Formel (II)**
wobei für den Rest R¹ die oben genannte Bedeutung und für den Rest X gilt:
X = Chlor, Brom oder lod,
(R²)₃Si-N=C=N-Si(R²)₃ **Formel (III)**
wobei für die Reste R² gleichzeitig oder unabhängig voneinander gilt,
R² = gleichzeitig oder unabhängig voneinander linear oder verzweigt C₁- bis C₅-Alkyl,
b) Hydrolysieren des in Schritt a) intermediär gebildeten, silylierten Magnesium-Carboxamidinat gemäß Formel (IV)
R¹-C(=N-Si(R²)₃)-N(-Si(R²)₃)·Mg-X **Formel (IV)**
wobei für die Reste R¹, R² und X die oben genannte Bedeutung gilt,
c) Isolieren der *N,N'*-nichtsubstituierten Carboxamidine der allgemeinen Formel (I) oder eines Salzes hiervon.

2. Verfahren nach Anspruch 1, **dadurch gekennzeichnet, dass** als organisches Lösungsmittel eingesetzt wird:
i) ein Ether, ausgewählt aus der Gruppe Diethylether, Di-n-butylether, t-Butyl-methylether, Dimethoxymethan, Diethoxymethan, 1,2-Dimethoxymethan, Tetrahydrofuran, 1,4-Dioxan, sowie Gemische hiervon, oder
ii) ein Kohlenwasserstoff, ausgewählt aus der Gruppe Petroleumfraktionen, n-Pentan, n-Hexan, n-Heptan, n-Octan, Isooctan, Cyclohexan, Methylcyclohexan, Benzol, Toluol oder Xylol, sowie Gemische hiervon, oder
iii) ein Gemisch aus i) und ii).

3. Verfahren nach mindestens einem der vorgenannten Ansprüche, **dadurch gekennzeichnet, dass** in Verfahrensstufe a) als *N*,*N*'-Bis(trialkylsilyl)carbodiimid gemäß Formel (III) *N*,*N'-*Bis(trimethylsilyl)carbodiimid eingesetzt wird.

4. Verfahren nach Anspruch 1 **dadurch gekennzeichnet, dass** die Umsetzung in Stufe a) bei einer Temperatur im Bereich von - 40 °C bis 180 °C und/oder bei einem Druck im Bereich von 0,01 bis 10.000 mbar durchgeführt werden.

5. Verfahren nach mindestens einem der vorgenannten Ansprüche, **dadurch gekennzeichnet, dass** in Verfahrensstufe a) pro Mol magnesiumorganische Verbindung gemäß Formel (II) 0,8 bis 1,2 Mol des *N,N'*-Bis(trialkylsilyl)carbodiimid gemäß Formel (III) eingesetzt wird.

6. Verfahren nach mindestens einem der vorgenannten Ansprüche, **dadurch gekennzeichnet, dass** in Verfahrensstufe b) als Hydrolysierungsmittel Wasser und/ oder eine Säure und/oder ein C₁- bis C₅-Alkohol eingesetzt wird.

7. Verfahren nach mindestens einem der vorgenannten Ansprüche, **dadurch gekennzeichnet, dass** in Verfahrensstufe b) das Hydrolysierungsmittel im 1- bis 10-fachen molaren Überschuss, bezogen auf die eingesetzte molare Menge der magnesiumorganischen Verbindung der allgemeinen Formel (II) einsetzt wird.

8. Verfahren nach mindestens einem der vorgenannten Ansprüche, **dadurch gekennzeichnet, dass** das in Verfahrensstufe b) als Hydrolysierungsmittel Salzsäure eingesetzt wird und/oder dass das *N*,*N*'-unsubstituierte Carboxamidin gemäß Formel (I) als Hydrochlorid isoliert wird.

9. Verfahren nach mindestens einem der vorgenannten Ansprüche, **dadurch gekennzeichnet, dass** die Isolierung in Verfahrensstufe c) durch Zugabe eines organischen, nicht wasserlöslichen Lösungsmittels mittels Extraktion und anschließendes Verdampfen des nicht wasserlöslichen Lösungsmittels erfolgt.

## Claims

1. Method for preparing *N,N'*-unsubstituted carboxamidines of general formula (I) or a salt thereof
R¹-C(=NH)-NH₂ **Formula (I)**
wherein the following applies to the group R¹:
R¹= linear or branched C₁ to C₂₀ alkyl, C₃ to C₂₀ cycloalkyl, C₄ to C₂₀ cycloalkylalkyl, C₆ to C₂₀ aryl, and C₇ to C₂₀ arylalkyl, wherein said groups can be mono- or polysubstituted and the substituents are selected from the group consisting of fluorine, chlorine, C₁ to C₈ alkyloxy, C₆ to C₈ aryloxy, and C₁ to C₈ dialkylamino,
the method comprising the following method steps:
a) reacting an organomagnesium compound of general formula (II) with an *N,N'-*bis(trialkylsilyl)carbodiimide according to formula (III) in an organic solvent,
wherein the following applies to formula (II) and formula (III):
R¹-Mg-X **Formula (II)**
wherein the above-mentioned definition applies to the group R¹ and the following applies to group X:
X = chlorine, bromine or iodine,
(R²)₃Si-N=C=N-Si(R²)₃ **Formula (III)**
wherein the following applies to the groups R², simultaneously or independently of one another,
R² = simultaneously or independently of one another, linear or branched C₁ to C₅ alkyl;
b) hydrolysing the silylated magnesium carboxamidinate, formed intermediately in step a), according to formula (IV)
R¹-C(=N-Si(R²)₃)-N(-Si(R²)₃)·Mg-X **Formula (IV)**
wherein the above-mentioned definition applies to the groups R¹, R² and X;
c) isolating the *N,N'*-unsubstituted carboxamidines of general formula (I) or a salt thereof.

2. Method according to claim 1, **characterised in that** the following is used as an organic solvent:
i) an ether selected from the group consisting of diethyl ether, di-n-butyl ether, t-butyl methyl ether, dimethoxymethane, diethoxymethane, 1,2-dimethoxymethane, tetrahydrofuran, 1,4-dioxane, and mixtures thereof; or
ii) a hydrocarbon selected from the group consisting of petroleum fractions, n-pentane, n-hexane, n-heptane, n-octane, isooctane, cyclohexane, methylcyclohexane, benzene, toluene or xylene, and mixtures thereof; or
iii) a mixture of i) and ii).

3. Method according to at least one of the preceding claims, **characterised in that**, in method stage a), N,N'-bis(trimethylsilyl)carbodiimide is used as *N,N'-*bis(trialkylsilyl)carbodiimide according to formula (III).

4. Method according to claim 1, **characterised in that** the reaction in stage a) is carried out at a temperature in the range of from -40°C to 180°C and/or at a pressure in the range of from 0.01 to 10,000 mbar.

5. Method according to at least one of the preceding claims, **characterised in that**, in method stage a), 0.8 to 1.2 moles of the *N,N'*-bis(trialkylsilyl)carbodiimide according to formula (III) is used per mole of organomagnesium compound according to formula (II).

6. Method according to at least one of the preceding claims, **characterised in that**, in method stage b), water and/or an acid and/or a C₁ to C₅ alcohol is used as the hydrolysing agent.

7. Method according to at least one of the preceding claims, **characterised in that**, in method stage b), the hydrolysing agent is used in a 1 to 10-fold molar excess, based on the molar amount of the organomagnesium compound of general formula (II) used.

8. Method according to at least one of the preceding claims, **characterised in that**, in method stage b), hydrochloric acid is used as the hydrolysing agent and/or that the *N,N'*-unsubstituted carboxamidine according to formula (I) is isolated as the hydrochloride.

9. Method according to at least one of the preceding claims, **characterised in that** the isolation in method stage c) is carried out by adding an organic, water-insoluble solvent by means of extraction and subsequent evaporation of the water-insoluble solvent.

## Revendications

1. Procédé de préparation de carboxamidines N,N'-non substituées de formule générale (I) ou d'un de leurs sels
R¹-C(=NH)-NH₂ **Formule (I)**
où le radical R¹ a la signification suivante :
R¹ = alkyle en C₁ à C₂₀ linéaire ou ramifiée, cycloalkyle en C₃ à C₂₀, cycloalkylalkyle en C₄ à C₂₀, aryle en C₆ à C₂₀, arylalkyle en C₇ à C₂₀,
ces radicaux pouvant en outre être substitués une ou plusieurs fois et les substituants étant choisis dans le groupe comprenant le fluor, le chlore, les groupes alkyloxy en C₁ à C₈, aryloxy en C₆ à C₈, dialkylamino en C₁ à C₈,
comprenant les étapes de procédé suivantes
a) faire réagir un composé organomagnésien de formule générale (II) avec un N,N'-bis(trialkylsilyl)carbodiimide selon la formule (III) dans un solvant organique, dans laquelle la formule (II) et la formule (II) sont les suivantes
R¹-Mg-X **Formule (II)**
où le radical R¹ a la signification ci-dessus et où le radical X a la signification suivante :
X = chlore, brome ou iode,
(R²)₃Si-N=C=N-Si(R²)₃ **Formule (III)**
où les radicaux R2 ont la signification suivante simultanément ou indépendamment l'un de l'autre,
R² = simultanément ou indépendamment l'un de l'autre un alkyle en C₁ à C₅, linéaire ou ramifié,
b) hydrolyser le carboxamidinate de magnésium silylé formé de façon intermédiaire à l'étape a) selon la formule (IV)
R¹-C(=N-Si(R2)₃)-N(-Si(R²)₃)·Mg-X **Formule (IV)**
où les radicaux R¹, R² et X ont la signification susmentionnée,
c) isoler les carboxamidines N,N'-non substituées de formule générale (I) ou un de leurs sels.

2. Procédé selon la revendication 1, **caractérisé en ce que** le solvant organique utilisé est :
i) un éther choisi dans le groupe comprenant l'éther diéthylique, l'éther di-n-butylique, l'éther t-butylméthylique, le diméthoxyméthane, le diéthoxyméthane, le 1,2-diméthoxyméthane, le tétrahydrofuranne, le 1,4-dioxane et leurs mélanges, ou
ii) un hydrocarbure choisi dans le groupe comprenant les fractions pétrolières, le n-pentane, le n-hexane, le n-heptane, le n-octane, l'isooctane, le cyclohexane, le méthylcyclohexane, le benzène, le toluène ou le xylène, et leurs mélanges ; ou
iii) un mélange de i) et ii).

3. Procédé selon au moins une des revendications précédentes, **caractérisé en ce que** dans l'étape de procédé a), on utilise comme *N,N'*-bis(trialkylsilyl)carbodiimide le N,N'-bis(triméthylsilyl)carbodiimide selon la formule (III).

4. Procédé selon la revendication 1, **caractérisé en ce que** la réaction de l'étape a) est réalisée à une température comprise entre -40°C et 180°C et/ou à une pression comprise entre 0,01 et 10.000 mbar.

5. Procédé selon au moins une des revendications précédentes, **caractérisé en ce que** dans l'étape de procédé a) on utilise 0,8 à 1,2 mole du *N,N'*-bis(trialkylsilyl)carbodiimide selon la formule (III) par mole de composé organomagnésien selon la formule (II).

6. Procédé selon au moins une des revendications précédentes, **caractérisé en ce que** de l'eau et/ou un acide et/ou un alcool en C₁ à C₅ sont utilisés comme agent hydrolysant dans l'étape de procédé b).

7. Procédé selon au moins une des revendications précédentes, **caractérisé en ce que** dans l'étape de procédé b), l'agent hydrolysant est utilisé en un excès molaire de 1 à 10 fois, par rapport à la quantité molaire du composé organomagnésien de formule générale (II) utilisé.

8. Procédé selon au moins une des revendications précédentes, **caractérisé en ce que** l'acide chlorhydrique est utilisé comme agent hydrolysant dans l'étape de procédé b) et/ou **en ce que** la carboxamidine N,N'-non substituée selon la formule (I) est isolé sous forme de hydrochlorure.

9. Procédé selon au moins une des revendications précédentes, **caractérisé en ce que** l'isolation dans l'étape de procédé c) est réalisée par l'addition d'un solvant organique pas soluble dans l'eau, par extraction et ensuite par évaporation du solvant pas soluble dans l'eau.
